Europäisches Patentamt

European Patent Office

Office européen des brevets

(19)

(11) Publication number: **0 243 200 B1**

(12) **EUROPEAN PATENT SPECIFICATION**

(45) Date of publication of patent specification: **08.07.92**   (51) Int. Cl.⁵: **C12Q 1/00**, C12Q 1/34

(21) Application number: **87303644.6**

(22) Date of filing: **24.04.87**

(54) **Method of measuring lipid-bound sialic acid and reagent kit for use therein.**

(30) Priority: **25.04.86 JP 94613/86**

(43) Date of publication of application:
**28.10.87 Bulletin 87/44**

(45) Publication of the grant of the patent:
**08.07.92 Bulletin 92/28**

(84) Designated Contracting States:
**CH DE FR GB IT LI NL**

(56) References cited:
**EP-A- 0 038 529**
**EP-A- 0 133 694**
**EP-A- 0 159 563**
**US-A- 4 115 062**

(73) Proprietor: **FUJIREBIO KABUSHIKI KAISHA**
**also trading as FUJIREBIO INC.**
**6-7, Shimoochiai 4-chome Shinjuku-ku**
**Tokyo 161(JP)**

(72) Inventor: **Sugiyama, Masami**
**2-1-306, Utsukidai 1067, Utsuki-cho**
**Hachioji-shi Tokyo(JP)**

(74) Representative: **Silverman, Warren et al**
**HASELTINE LAKE & CO. Hazlitt House 28**
**Southampton Buildings Chancery Lane**
**London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

This invention relates to a method of measuring lipid-bound sialic acid (LSA) and to a reagent kit for use therein.

Recently, there have been many reports that when persons suffer from cancer, LSA concentration in their blood increases. Indeed, it has also been reported that, even in a patient having primary gastric cancer and whose total sialic acid concentration has not increased, LSA concentration in the blood of the patient has significantly increased (Oki, S., J. Jpn. Soc. Cancer Ther., vol. l8, pp 692-703 (l983)).

The method of measuring LSA developed by N. Katopodis et al. (Res. Commun. Clin. Path. Pharm., vol. 30, pp l7l-l80 (l980)) has been widely employed. This method comprises the following steps. First, a sample of test serum is pipetted into a test tube, and distilled water is added. The mixture is cooled to 4°C, and an extractant (chloroform : methanol = 2:l by vol.) is added. This mixture is vigorously stirred for 30 seconds, and centrifuged at ambient temperature. The supernatant is put into another test tube, and an aqueous phosphotungstic acid solution is added to precipitate LSA. The precipitate is collected by centrifuging, and dissolved in distilled water. A resorcinol reagent is added to the solution, and allowed to react at l00°C for 20 minutes. After cooling to ambient temperature, a solvent mixture of butyl acetate-butanol is added to extract a coloured material. The absorbance of the butyl acetate-butanol layer is determined as a measure of the LSA concentration of the sample.

As will be appreciated from the foregoing, the method of Katopodis et al. is complicated and hence an enzyme method has been developed (Japanese Patent KOKAI No.60-78597). In this method, the sample is divided into two parts after it has been subjected to pretreatments such as dilution and solvent extraction of free fatty acids. One part is kept for measuring total sialic acid, and phosphotungstic acid is added to the other part to precipitate LSA. The sialic acid concentration of both the part not treated with phosphotungstic acid and the supernatant of the other part treated with phosphotungstic acid are measured by the enzyme method such as developed by Sugahara et al (Clin. Chim. Acta, vol. l08, pp 493-498 (l980) wherein neuraminidase and N-acetylneuraminic acid aldolase are employed. The LSA concentration of the sample is determined by subtraction of the above two sialic acid concentrations. This method is simpler than the previously mentioned method. However, this method does not yield directly LSA concentrations, these being indirectly determined by subtraction.

It is an object of the invention to provide a method of measuring LSA which is simple to carry out but which yields measured values which are exact and highly reliable. The method should provide reproducibility. More particularly, this invention should provide a LSA separating agent whose performance in the separation of other forms of sialic acid such as protein-bound sialic acid, from LSA is good and which is suitable for use in measuring LSA using the foregoing enzyme method.

According to the present invention, there is firstly provided a method of measuring lipid-bound sialic acid which comprises contacting a separating agent for lipid-bound sialic acid, the separating agent comprising a polar solvent, with a sample containing lipid-bound sialic acid and one or more other forms of sialic acid, separating a supernatant from a precipitate formed, reacting said supernatant with an enzyme capable of acting on sialic acid in a lipid-bound state to yield a determinable molecular fragment and determining said fragment as a measure of the lipid-bound sialic acid in said sample.

This invention also provides a reagent kit for use in measuring lipid-bound sialic acid, which kit comprises, as separate components, a polar solvent-comprising lipid-bound sialic acid separating agent, an enzyme reagent which acts on lipid-bound sialic acid to produce a determinable molecular fragment and a reagent for reacting with said fragment to yield a colourimetrically determinable compound as a measure of lipid-bound sialic acid in a test sample.

This invention is based on the following discoveries. Firstly, when samples containing sialic acid in various states are treated with a LSA separating agent comprising a polar solvent, the sialic acids other than LSA precipitate while LSA remains in the supernatant. That is, LSA can easily be separated without complicated operations. Secondly, LSA in the supernatant can be determined by the enzyme method developed by Sugahara et al without any special additional operating steps, and the results are then consistent with those obtained in the conventional method of Katopodis et al.

The LSA separating agent comprises a polar solvent. Suitable polar solvents include methanol, ethanol, propanol, isopropanol, dimethylformamide and acetonitrile. Two or more polar solvents may be used in combination. The LSA separating agent may be constituted of polar solvent(s) alone, or it may contain another component. For example, a small amount of water or an aqueous solution of a protein precipitant such as heparin may be added to a polar solvent. In this case, the polar solvent preferably constitutes more than 90 vol.%.

The amount of LSA separating agent to be added is determined mainly by the protein content of each

2

test sample. When the sample is serum, the volume of the protein precipitant is preferably l.2 to l.6 times the volume of the sample. The reaction time for the LSA separating agent to react with the sample may be 5 to 30 seconds at ambient temperature. The precipitate formed is separated by filtration or centrifuging. Filtration using a tube filter is preferable for purposes of automation of operation.

The sialic acid concentration of the supernatant is measured by using an enzyme capable of acting on sialic acid in a lipid-bound state. Such enzymes includes neuraminidase and N-acetylneuraminic acid aldolase. In this method, sialic acid existing in a lipid-bound state is liberated by neuraminidase to produce N-acetylneuraminic acid which is a free sialic acid, and the N-acetylneuraminic acid is decomposed by N-acetylneuraminic acid aldolase to N-acetylmannosamine and pyruvic acid. Then, the amount of the pyruvic acid may be determined by a known method. For example, the pyruvic acid is oxidised by pyruvate oxidase to produce $H_2O_2$, and $H_2O_2$ is allowed to react with 4-aminoantipyrine and an oxidative condensing agent such as N-ethyl-N-($\beta$-hydroxyethyl)-m-toluidine in the presence of peroxidase to produce a red to violet colour. Then, the intensity of this colour is determined by measuring absorbance at 550 nm. LSA concentration can be determined for example, by means of a graph in which absorbance is plotted against concentration. A reagent kit for this enzyme determination, as such, is commercially available. Alternatively, lactate dehydrogenase is allowed to react with the above pyruvic acid in the presence of NADH as a coenzyme, and the decrease in concentration of the NADH may be determined by measuring absorbance at 340-370 nm. LSA concentration can also be determined from the result obtained in equivalent manner.

When a test sample is treated with a LSA separating agent comprising a polar solvent, various components in the sample such as protein-bound sialic acid and proteinaceous substances precipitate while the object lipid-bound sialic acid remains in the supernatant. Accordingly, by measuring the sialic acid concentration of the supernatant, the LSA concentration of the test sample is easily determined. The method of the invention is simple to carry out and highly reproducible. The method of the invention is suitable for treating a large number of samples, and it can easily be automated. This method is suitable for use in screening for cancer.

The following examples illustrate this invention:

EXAMPLE I

95 parts by volume of a solvent mixture of methanol/dimethylformamide (l/l v/v) were mixed with 5 parts by volume of distilled water, and used as a LSA separating agent. In each test, l50 $\mu$l of this LSA separating agent were added to l00 $\mu$l of sample serum, and vigorously stirred by a mixer for l0 seconds. The precipitate which formed was separated by a centrifuge at 3,000 rpm (more than l,000 G) to obtain l50 $\mu$l of supernatant.

l50 $\mu$l each of the supernatant separated from a sample serum and from a standard serum were placed into separate small test tubes, and l ml of the enzyme colouring solution comprised by the reagent kit for measuring sialic acid ("SIALIZYME-550", Fujirebio Inc.) was added to each test tube. This enzyme colouring solution contained neuraminidase, N-acetylneuraminic acid aldolase, 4-aminoantipyrine, pyruvate oxidase, peroxidase, N-ethyl-N-( $\beta$-hydroxyethyl)-m-toluidine, $MgCl_2$, FAD, TPP and other substances. This mixture was allowed to react at 37°C for 20 minutes, and 2 ml of a reaction termination solution which was also present in the above reagent kit was added. Then, the absorbance of the mixture of 550 nm was measured. At this stage, l50 $\mu$l of distilled water were employed, instead of the sample serum, as reagent blank.

The LSA concentration of the sample was calculated by means of the following formula.

$$\text{LSA (mg/dl} = \frac{\text{Absorbance of Sample}}{\text{Absorbance of Standard}} \times \text{Sialic Acid}$$

Concentration of Standard $\times$ Extent of dilution of sample (when l00 $\mu$l of a sample is mixed with l50 $\mu$l of a LSA separating agent, the extent of dilution is 2.5.

In order to examine reproducibility, the above measurement was repeated l0 times with each of two sample sera. The results are shown below.

EP 0 243 200 B1

| n=O | Sample A | Sample B |
|---|---|---|
| $X_{max}$ | 13.4 mg/dl | 21.9 mg/dl |
| $X_{min}$ | 12.6 mg/dl | 20.9 mg/dl |
| $\bar{x}$ | 13.2 mg/dl | 21.3 mg/dl |
| SD | 0.32 | 0.34 |
| CV | 2.4% | 1.6% |

CV: Coefficient of Variation

By experimenting with 26 sample sera, it was possible to achieve correlation between the results obtained by the above measurement and the results obtained by the conventional Katopodis method. The results are shown below.

n = 26
Correlation coefficient r = 0.95
Regression formula Y = I.0X - 0.5 (mg/dl)
Y: The Present Method
X: Conventional Method

EXAMPLE 2

A mixture of 97 parts by volume of methanol and 3 parts by volume of distilled water was used as the LSA separating agent, and the LSA concentrations of the two samples (Sample A and Sample B) were measured in the same manner as in Example I. Measurement was repeated each I0 times, and the results are shown below.

| n=10 | Sample A | Sample B |
|---|---|---|
| $X_{max}$ | 13.6 mg/dl | 22.4 mg/dl |
| $X_{min}$ | 13.1 mg/dl | 21.1 mg/dl |
| $\bar{x}$ | 13.4 mg/dl | 21.7 mg/dl |
| SD | 0.20 | 0.53 |
| CV | 1.5% | 2.4% |

Correlation to Conventional Katopodis Method
n = 26
Correlation coefficient r = 0.96
Regression formula Y = I.0IX + 0.7 (mg/dl)

EXAMPLE 3

A mixture of 95 parts by volume of dimethylformamide/isopropanol (60/40 v/v) and 5 parts by volume of distilled water was used as the LSA separating agent and the LSA concentrations of the aforesaid samples A and B were measured in the same manner as Example I. Measurement was repeated I0 times, and the results are shown below.

4

| n=10 | Sample A | Sample B |
|---|---|---|
| $X_{max}$ | 13.9 mg/dl | 21.8 mg/dl |
| $X_{min}$ | 12.7 mg/dl | 20.4 mg/dl |
| $\bar{x}$ | 13.5 mg/dl | 21.4 mg/dl |
| SD | 0.50 | 0.67 |
| CV | 3.7% | 3.1% |

Correlation to Conventional Katopodis Method
n = 26
Correlation coefficient r = 0.96
Regression formula Y = 0.97X + 0.9 (mg/dl)

EXAMPLE 4

Working with a further sample serum, measurement of LSA concentration was carried out both by the conventional Katopodis method, and the reproducibilities of the measurements obtained were compared to each other. The results are shown below.

| n=10 | The Present Method | Conventional Method |
|---|---|---|
| $X_{max}$ | 12.0 mg/dl | 11.5 mg/dl |
| $X_{min}$ | 10.9 mg/dl | 8.6 mg/dl |
| $\bar{x}$ | 11.7 mg/dl | 10.6 mg/dl |
| SD | 0.41 | 0.90 |
| CV | 3.5% | 8.5% |

**Claims**

1. A method of measuring lipid-bound sialic acid which comprises contacting a separating agent for lipid-bound sialic acid, the separating agent comprising a polar solvent, with a sample containing lipid-bound sialic acid and one or more other forms of sialic acid, separating a supernatant from a precipitate formed, reacting said supernatant with an enzyme capable of acting on sialic acid in a lipid-bound state to yield a determinable molecular fragment and determining said fragment as a measure of the lipid-bound sialic acid in said sample.

2. The method of claim I wherein said separating agent consists of or contains a single polar solvent.

3. The method of claim I wherein said separating agent consists of or contains a mixture of a polar solvent and water, the content of said polar solvent being more than 90 vol.%.

4. The method of claim I wherein said polar solvent consists of or contains a mixture of two or more polar solvents.

5. The method of claim 2, 3 or 4, wherein a said polar solvent is selected from methanol, ethanol, propanol, isopropanol, dimethylformamide and acetonitrile.

6. The method of any preceding claim, wherein said separating agent contains a protein precipitant.

7. The method of claim 6, wherein said protein precipitant is heparin.

**8.** The method of any preceding claim, wherein said sample is serum and the volume of said separating agent is l.2 to l.6 times the volume of serum being tested.

**9.** The method of any preceding claim, wherein said enzyme is neuraminidase and/or N-acetylneuraminic acid aldolase.

**10.** A reagent kit for use in measuring lipid-bound sialic acid, which kit comprises, as separate components, a polar solvent-comprising lipid-bound sialic acid separating agent, an enzyme reagent which acts on lipid-bound sialic acid to produce a determinable molecular fragment and a reagent for reacting with said fragment to yield a colourimetrically determinable compound as a measure of lipid-bound sialic acid in a test sample.

**11.** The reagent kit of claim 10 wherein said polar solvent is selected from methanol, ethanol, propanol, isopropanol, dimethylformamide and acetonitrile.

**12.** The reagent kit of claim 10 or 11, wherein the lipid-bound sialic acid separating agent is a mixture of a polar solvent and water in which the content of said polar solvent is more than 90 vol.%.

**13.** The reagent kit of any one of claims 10 to 12, in which the lipid-bound sialic acid separating agent contains a protein precipitant.

**14.** The reagent kit of claim 13 wherein said protein precipitant is heparin.

**Revendications**

**1.** Procédé pour déterminer l'acide sialique lié aux lipides, comprenant la mise en contact d'un agent séparateur de l'acide sialique lié aux lipides, l'agent séparateur comprenant un solvant polaire, avec un échantillon contenant de l'acide sialique lié aux lipides et une ou plusieurs autre(s) forme(s) de l'acide sialique, la séparation d'un surnageant d'avec le précipité formé, la réaction dudit surnageant avec une enzyme capable d'agir sur l'acide sialique à l'état lié aux lipides pour donner un fragment moléculaire déterminable, et la détermination dudit fragment comme mesure de l'acide sialique lié aux lipides dans ledit échantillon.

**2.** Procédé selon la revendication 1, dans lequel ledit agent séparateur est constitué par, ou contient un seul solvant polaire.

**3.** Procédé selon la revendication 1, dans lequel ledit agent séparateur est constitué par, ou contient un mélange d'un solvant polaire et d'eau, la teneur en ledit solvant polaire étant supérieure à 90 % en volume.

**4.** Procédé selon la revendication 1, dans lequel ledit solvant polaire est constitué par, ou contient un mélange de deux ou plus de deux solvants polaires.

**5.** Procédé selon la revendication 2, 3 ou 4, dans lequel ledit solvant polaire est choisi parmi le méthanol, l'éthanol, le propanol, l'isopropanol, le diméthylformamide et l'acétonitrile.

**6.** Procédé selon l'une quelconque des revendications précédentes, dans lequel ledit agent séparateur contient un précipitant des protéines.

**7.** Procédé selon la revendication 6, dans lequel ledit précipitant des protéines est l'héparine.

**8.** Procédé selon l'une quelconque des revendications précédentes, dans lequel ledit échantillon est du sérum, et le volume dudit agent séparateur est de 1,2 à 1,6 fois le volume du sérum à tester.

**9.** Procédé selon l'une quelconque des revendications précédentes, dans lequel ladite enzyme est la neuraminidase et/ou l'aldolase de l'acide N-acétylneuraminique.

**10.** Trousse de réactifs destinée à l'utilisation pour la détermination de l'acide sialique lié aux lipides, qui comprend, sous forme de composants séparés, un agent séparateur de l'acide sialique lié aux lipides, comprenant un solvant polaire, un réactif à base d'enzyme qui agit sur l'acide sialique lié aux lipides pour donner un fragment moléculaire déterminable, et un réactif pour réagir avec ledit fragment pour donner un composé déterminable par colorimétrie, comme mesure de l'acide sialique lié aux lipides dans un échantillon à tester.

11. Trousse de réactifs selon la revendication 10, dans laquelle ledit solvant polaire est choisi parmi le méthanol, l'éthanol, le propanol, l'isopropanol, le diméthylformamide et l'acétonitrile.

12. Trousse de réactifs selon la revendication 10 ou 11, dans laquelle l'agent séparateur de l'acide sialique lié aux lipides est un mélange d'un solvant polaire et d'eau, dont la teneur en ledit solvant polaire est supérieure à 90 % en volume.

13. Trousse de réactifs selon l'une quelconque des revendications 10 à 12, dans laquelle l'agent séparateur de l'acide sialique lié aux lipides contient un précipitant des protéines.

14. Trousse de réactifs selon la revendication 13, dans laquelle ledit précipitant des protéines est l'héparine.

**Patentansprüche**

1. Verfahren zur Messung von lipidgebundener Sialinsäure, welches umfaßt das Inberührungbringen eines ein polares Lösungsmittel enthaltenden Trennmittels für die lipidgebundene Sialinsäure mit einer Probe, die lipidgebundene Sialinsäure und eine oder mehrere andere Formen von Sialinsäure enthält, das Abtrennen einer überstehenden Flüssigkeit von einem gebildeten Präzipitat, das Reagierenlassen der überstehenden Flüssigkeit mit einem Enzym, das geeignet ist für die Einwirkung auf Sialinsäure in einem lipidgebundenen Zustand unter Bildung eines meßbaren Molekülfragments, und die Bestimmung des Molekülfragments als Maß für die lipidgebundene Sialinsäure in der Probe.

2. Verfahren gemäß Anspruch 1, wobei das Trennmittel besteht aus oder enthält ein einziges polares Lösungsmittel.

3. Verfahren gemäß Anspruch 1, wobei das Trennmittel besteht aus oder enthält eine Mischung aus einem polaren Lösungsmittel und Wasser und der Gehalt an dem polaren Lösungsmittel mehr als 90 Vol.-% beträgt.

4. Verfahren gemäß Anspruch 1, wobei das polare Lösungsmittel besteht aus oder enthält eine Mischung von zwei oder mehr polaren Lösungsmitteln.

5. Verfahren gemäß Anspruch 2, 3 oder 4, wobei das polare Lösungsmittel ausgewählt ist aus Methanol, Ethanol, Propanol, Isopropanol, Dimethylformamid und Acetonitril.

6. Verfahren gemäß einem jeden der vorangehenden Ansprüche, wobei das Trennmittel ein Protein-Fällungsmittel enthält.

7. Verfahren gemäß Anspruch 6, wobei das Protein-Fällungsmittel Heparin ist.

8. Verfahren gemäß einem jeden der vorangehenden Ansprüche, wobei die Probe Serum ist und das Volumen des Trennmittels das 1,2 bis 1,6-fache des Volumens vom zu untersuchenden Serum ist.

9. Verfahren gemäß einem jeden der vorangehenden Ansprüche, wobei das Enzym Neuraminidase und/oder N-Acetylneuraminsäure-Aldolase ist.

10. Reagenzsatz zum Einsatz für die Messung von lipidgebundener Sialinsäure, enthaltend als getrennte Komponenten ein ein polares Lösungsmittel umfassendes Trennmittel für die lipidgebundene Sialinsäure, ein Enzymreagenz, welches auf die lipidgebundene Sialinsäure unter Bildung eines meßbaren Molekülfragments einwirkt, sowie ein Reagenz zur Einwirkung auf das Fragment unter Bildung einer colorimetrisch bestimmbaren Verbindung als Maß für die lipidgebundene Sialinsäure in der Probe.

11. Reagenzsatz gemäß Anspruch 10, wobei das Lösungsmittel ausgewählt ist aus Methanol, Ethanol, Propanol, Isopropanol, Dimethylformamid und Acetonitril.

12. Reagenzsatz gemäß Anspruch 10 oder 11, wobei das Trennmittel für die lipidgebundene Sialinsäure aus einer Mischung von einem polaren Lösungsmittel und Wasser mit einem Gehalt an dem polaren Lösungsmittel von mehr als 90 Vol.-% besteht.

13. Reagenzsatz gemäß einem jeden der Ansprüche 10 bis 12, wobei das Trennmittel für die lipidgebundene Sialinsäure ein Protein-Fällungsmittel enthält.

14. Reagenzsatz gemäß Anspruch 13, wobei das Protein-Fällungsmittel Heparin ist.